(19) **Europäisches Patentamt / European Patent Office / Office européen des brevets**

(11) **EP 3 599 886 B1**

(12) **FASCICULE DE BREVET EUROPEEN**

(45) Date de publication et mention
de la délivrance du brevet:
**03.03.2021 Bulletin 2021/09**

(21) Numéro de dépôt: **18713954.8**

(22) Date de dépôt: **29.03.2018**

(51) Int Cl.:
*A23K 20/163* (2016.01)  *A61K 9/113* (2006.01)
*A23K 50/60* (2016.01)  *A23L 29/10* (2016.01)
*A23L 35/00* (2016.01)

(86) Numéro de dépôt international:
**PCT/EP2018/058141**

(87) Numéro de publication internationale:
**WO 2018/178270 (04.10.2018 Gazette 2018/40)**

(54) **GLUCOSE ENCAPSULÉ DANS UNE COMPOSITION DE REMPLACEMENT DE LAIT POUR LES VEAUX**

IN EINER MILCHERSATZZUSAMMENSETZUNG FÜR KÄLBER VERKAPSELTE GLUCOSE

GLUCOSE ENCAPSULATED IN A MILK REPLACEMENT COMPOSITION FOR CALVES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.03.2017 BE 201705208**

(43) Date de publication de la demande:
**05.02.2020 Bulletin 2020/06**

(73) Titulaire: **Tereos Starch & Sweeteners Belgium 9300 Aalst (BE)**

(72) Inventeurs:
• **APPER, Emmanuelle**
**82220 Labarthe (FR)**
• **REDL, Andreas**
**77230 Moussy-le-Vieux (FR)**
• **SANDOU, Nancy**
**78955 Carrières-sous-Poissy (FR)**
• **SAUREL, Rémi**
**21078 Dijon Cedex (FR)**

(74) Mandataire: **Icosa**
**83 avenue Denfert-Rochereau**
**75014 Paris (FR)**

(56) Documents cités:
**WO-A1-2009/003960    WO-A1-2015/197089**
**FR-A1- 2 766 737    FR-A1- 2 828 378**
**JP-A- H0 523 133    US-A- 5 756 132**

**Description**

**DOMAINE DE L'INVENTION**

La présente invention concerne une émulsion double E1/H/E2 encapsulant du glucose, des compositions comprenant ladite émulsion, notamment des compositions de remplacement du lait. L'invention concerne également, la méthode d'obtention d'une telle composition.

**ÉTAT DE LA TECHNIQUE**

Les veaux sont couramment nourris avec un lait de remplacement, une portion de fourrage et de concentré énergétique et/ou protéique. Le lactose est la principale source de glucides des aliments complets d'allaitement de veau de boucherie. Sa présence est due à l'ajout de poudre de lactosérum à hauteur de 78 % de la matière sèche. Ainsi, les laits de remplacement contiennent un taux élevé de lactose, très digestible pour le veau, mais dont la volatilité des prix oblige les fabricants d'aliments à tester des alternatives. L'amidon issu des végétaux comme les céréales, les tubercules ou les légumineuses ainsi que les dérivés de l'amidon telles que les maltodextrines ou les dextrines sont aussi considérés comme des sources potentielles de glucides mais nécessitent pour leur métabolisation la présence d'amylase (*Gautier & Labussière, 2006*). Or, le jeune veau possède un système enzymatique très limité pour digérer les glucides complexes autres que le lactose. Dès lors, l'intérêt de l'utilisation d'amidon et de dérivés d'amidon dans le remplacement du lactose semble limité (*Gautier & Labussière, 2006*).

Le glucose est à l'inverse très digestible. Cependant, il peut, s'il est ingéré dans des quantités massives, induire des désordres métaboliques et notamment des dérèglements de l'homéostasie glucidique et des diarrhées. Ainsi, bien que représentant un candidat d'intérêt, l'ajout massif de glucose dans l'aliment complet d'allaitement du veau semble de fait non applicable.

Par ailleurs, la composition des lactoremplaceurs destinés à la nutrition animale devant être conforme à la réglementation en vigueur, le choix parmi les ingrédients et additifs autorisé est limité.

Il y a donc un besoin non satisfait d'un lait de remplacement pour les mammifères et notamment le veau comprenant une composition pouvant substituer le lait lactoremplaceur qui est hautement digestible, d'un coût limité, et qui ne présente pas d'effet délétère pour le métabolisme ou le système gastrointestinal de l'animal.

De manière surprenante, la double émulsion selon la présente invention, permet la libération avantageuse du glucose. Cette libération contrôlée permet l'administration des compositions comprenant la double émulsion chez les sujets caractérisés par des troubles gastrointestinaux ou métaboliques.

FR 2 828 378 A1 divulgue une émulsion multiple alimentaire et son procédé de préparation.

WO 2015/197089 A1 divulgue une composition de remplacement du lait, comprenant du glucose.

**RÉSUMÉ**

La Demanderesse a développé une double émulsion qui est stable et qui permet de véhiculer du glucose. Avantageusement, le glucose est libéré de manière contrôlée de la double émulsion, une fois en contact avec le milieu gastrique du sujet qui l'a ingérée. L'invention concerne donc une double émulsion eau-huile-eau, qui comprend une phase aqueuse interne E1. Cette phase aqueuse interne E1 comprend du glucose de : 31 70% w/w, de préférence de 35 à 55% w/w en poids par rapport à la masse d'E1 ; une phase huileuse H, comprenant une matière grasse animale et/ou végétale, et un émulsifiant lipophile ; et une phase aqueuse externe E2, comprenant un émulsifiant hydrosoluble. Le ratio massique de la phase aqueuse interne E1 sur la phase huileuse H étant de 7 : 3 à 5 : 5.

Dans un mode de réalisation, l'émulsifiant hydrosoluble est choisi parmi les caséinates, les protéines de blé hydrolysées, préférentiellement, l'agent émulsifiant hydrosoluble est le sodium caséinate. Cet émulsifiant hydrosoluble est de 6% à 12% w/w, de préférence de 8 à 10% w/w en poids par rapport à la masse de la phase externe E2.

Dans un mode de réalisation, l'émulsifiant lipophile est choisi parmi les monoglycérides, les diglycérides, la lécithine, les esters de sorbitane, les esters de saccharose d'acides gras, les esters de polyglycérol tel que le polyglycérol polyricinoléate et leurs combinaisons ; de préférence l'émulsifiant lipophile est le polyglycérol polyricinoléate.

La phase huileuse H selon l'invention est une matière grasse végétale choisie parmi de l'huile de palme, l'huile de germe de maïs, l'huile de tournesol, l'huile de colza, l'huile d'olive, l'huile de coprah et leur mélange.

Dans un mode de réalisation, la phase aqueuse extérieure E2 comprend en outre des protéines végétales choisies parmi des protéines de légumineuses, céréales et/ou oléagineuses ; et/ou des protéines végétales hydrolysées préférentiellement des protéines de blé hydrolysées.

Dans un mode de réalisation, la phase aqueuse extérieure E2 comprend en outre de la poudre de lactosérum doux et/ou de la poudre de lait écrémé.

Dans un mode de réalisation, la double émulsion selon l'invention est liquide, présentant une viscosité d'environ 10 à environ 100 mPa s à 25°C.

Dans la double émulsion selon l'invention, la phase aqueuse interne E1 est dispersée dans la phase huileuse H sous forme de gouttelettes. Les gouttelettes présentent un diamètre moyen d'environ 4 à environ 10 microns ou de préférence d'environ 6 à environ 8 microns.

L'invention concerne également, des compositions comprenant la double émulsion telle que décrite précédemment.

Dans un mode de réalisation, la composition comprend :

- des protéines, de préférence des protéines végétales et/ou animales, dans une concentration de 2 à 10 % w/w par rapport au poids de la composition ;
- des lipides, préférentiellement choisis parmi des matières grasses animales et/ou végétales, dans une concentration de 1 à 14 % w/w par rapport au poids de la composition ; et
- des minéraux, dans une concentration de 3 à 4 % w/w par rapport au poids de la composition.

Dans un mode de réalisation, les lipides de la composition sont dans une concentration de 1 à 5 % w/w par rapport au poids de la composition.

Les compositions de la présente invention sont notamment des compositions nutritionnelles permettant la substitution du lactose par le glucose. Avantageusement, les compositions comprenant la double émulsion de l'invention libèrent progressivement et de manière contrôlée le glucose dans le milieu gastrique.

L'invention concerne par conséquent la composition telle que décrite précédemment, pour son utilisation en tant que composition nutritionnelle pour un animal, de préférence un mammifère. Le mammifère peut être un ruminant, préférentiellement un ruminant néonatal, plus préférentiellement un bovin néonatal.

L'invention concerne également un procédé de préparation d'une double émulsion ou d'une composition de l'invention. Ce procédé comprend les étapes de :

a) préparer une phase aqueuse E1, ladite phase aqueuse E1 comprenant du glucose de 31 à 70% w/w, de préférence de 35 à 55% w/w en poids par rapport à la masse d'E1 ;

b) préparer une émulsion primaire E1/H, ladite émulsion primaire E1/H comprend la phase aqueuse E1 obtenue à l'étape (a), avec une phase huileuse H, ladite phase huileuse H comprenant une matière grasse animale et/ou végétale, et un émulsifiant lipophile; de préférence la préparation de l'émulsion primaire E1/H comprend la soumission de l'émulsion E1/H à une agitation de 20000 à 30000 tours/minute ; et

c) incorporer l'émulsion primaire E1/H obtenue à l'étape (b) dans une phase aqueuse E2, ladite phase aqueuse E2 comprenant un émulsifiant hydrosoluble.

## DESCRIPTION DÉTAILLÉE

L'invention concerne une double émulsion eau-huile-eau E1/H/E2 dans laquelle E1 et E2 sont des phases aqueuses et H est une phase lipidique, et dans laquelle émulsion la phase E1 comprend des molécules de glucose. La phase dite interne E1 est dispersée au sein de la phase lipidique H formant une émulsion E1/H, dite émulsion primaire. L'émulsion primaire E1/H est dispersée dans une deuxième phase aqueuse, dite phase externe E2.

Selon un mode de réalisation, la phase aqueuse interne E1, comprend du glucose :

- de 31 à 70% w/w ;
- de 35 à 60% w/w ;
- de 35 à 70% w/w ;
- de 35 à 60% w/w ;
- de 31 à 55% w/w ; ou
- de 35 à 55% w/w;

en poids par rapport à la masse d'E1.

Dans un mode de réalisation, la double émulsion eau-huile-eau E1/H/E2 comprend :

- une phase aqueuse interne E1, comprenant du glucose de 31 à 70% w/w, de préférence de 35 à 55% w/w en poids par rapport à la masse d'E1 ; et
- une phase aqueuse externe E2, comprenant un émulsifiant hydrosoluble.

Les phases E1 et H sont dans un ratio (w/w) de 7 :3 à 5 :5, avantageusement 6 :4

Le ratio de l'émulsion primaire (E1/H) par rapport à phase aqueuse externe E2 est dans un ratio émulsion primaire/ E2 (w/w) de 1 :9 à 8 :2 préférentiellement de 2 :8 à 7 :3, typiquement de 3 :7 à 4 :6.

Par conséquent, la phase aqueuse externe E2 est de 20-90%, 30-80% ou 60-70% (w/w) en poids par rapport à la masse de la double émulsion.

La phase aqueuse interne E1 est de 3-72%, 6-64%, 9-36%, 4-64%, 6-56%, 12-32%, 5-56%, 10-79%, 9-32%, 6-48%, 12-42%, 18-24% (w/w) en poids par rapport à la masse de la double émulsion.

La phase huileuse H est de 1-56%, 2-49%, 3-28%, 2-48%, 4-48%, 6-24%, 3-40%, 6-35%, 9-20%, 4-32%, 8-32% ou 12-16% (w/w) en poids par rapport à la masse de la double émulsion.

Selon l'invention, la double émulsion eau-huile-eau E1/H/E2 comprend :

- une phase aqueuse interne E1, comprenant du glucose de 31 à 70% w/w, de 35 à 65 % w/w ou de 35 à 60 % w/w, de préférence de 35 à 55% w/w en poids par rapport à la masse d'E1 ;
- une phase huileuse H, comprenant une matière grasse animale et/ou végétale, et un émulsifiant lipophile ;
- le ratio massique de la phase aqueuse interne E1 sur la phase huileuse H étant de 7 : 3 à 5 : 5 ; et
- une phase aqueuse externe E2, comprenant un émulsifiant hydrosoluble.

Dans un mode de réalisation, la double émulsion eau-huile-eau E1/H/E2 comprend :

- une phase aqueuse interne E1, comprenant du glucose de 31 à 70% w/w, de 35 à 65 % w/w ou de 35 à 60 % w/w, de préférence de 35 à 55% w/w en poids par rapport à la masse d'E1 ;
- une phase huileuse H, comprenant une matière grasse animale et/ou végétale, et un émulsifiant lipophile ;
- le ratio massique de la phase aqueuse interne E1 sur la phase huileuse H étant de 7 : 3 à 6 : 4 ou de 6 : 4 à 5 : 5, de préférence environ 6 : 4 ; et
- une phase aqueuse externe E2, comprenant un émulsifiant hydrosoluble.

« environ », placé devant un nombre, signifie plus ou moins 10% de la valeur nominale de ce nombre.

L'invention concerne également, une composition comprenant la double émulsion selon l'invention. Dans un mode de réalisation, la composition est une composition nutritionnelle pour un sujet. Dans un mode de réalisation, le sujet est un être humain sain ou un être humain ayant des troubles métaboliques (tels que le diabète, l'état prédiabétique, l'obésité, l'intolérance au glucose et/ou lactose) et/ou gastro-intestinaux (états inflammatoires gastrointestinaux, reflux gastriques etc.).

Selon un mode de réalisation, l'invention propose l'utilisation dans une composition de remplacement du lait, de glucose encapsulé dans une émulsion double E1/H/E2 dans laquelle E1 et E2 sont des phases aqueuses et H est une phase lipidique, et dans laquelle la phase E1 comprend des molécules de glucose.

L'expression "composition de remplacement du lait", telle qu'utilisée ici, comprend non seulement des substituts de lait produits entièrement à partir de matériaux isolés, mais également des substituts de lait fabriqués à partir de substituts dérivés de lait commerciaux ou d'autres substituts génériques de lait, Poudre (sèche) ou liquide (sirop).

Par « émulsion double E1/H/E2 » on entend une émulsion Eau-dans-Huile-dans-Eau E/H/E où E1 est la phase aqueuse interne renfermant le composé hydrosoluble à encapsuler et notamment le glucose et E2 la phase aqueuse externe (Gharsallaoui, 2009, Benichou et al., 2004). Dans ce type d'émulsion, il y a deux couches interfaciales différentes : une interface pour stabiliser l'émulsion E1/H et une autre pour la liaison H/E2. Par conséquent, sont utilisés un émulsifiant lipophile pour stabiliser les gouttelettes d'eau et un émulsifiant hydrosoluble pour stabiliser les globules d'huile. L'émulsion double E1/H/E2 peut être obtenue selon les méthodes décrites dans l'art antérieur (Gharsallaoui, 2009, Benichou et al., 2004).

Typiquement,

- la composition E1 est une composition aqueuse comprenant de 31 à 70%(w/w) de glucose, typiquement de 35 à 55% (w/w) de glucose ;
- la composition E2 est une composition aqueuse comprenant en outre, des protéines de lait plus particulièrement des caséinates et/ou des albumines et par exemple de la poudre de lactosérum et/ou de la poudre de lait écrémé, préférentiellement, la composition E2 comprend en outre des protéines végétales ;
- la composition H est choisie parmi les matières grasses animales et/ou végétales, la composition H comprend en outre un émulsifiant liposoluble.

L'une quelconque des compositions E1 et/ou E2 comprend au moins un agent émulsifiant hydrosoluble.

Dans un mode de réalisation, l'agent émulsifiant hydrosoluble ou le mélange d'agents émulsifiant hydrosolubles présente une valeur de balance Hydrophile-Lipophile (HLB) de 7 à 18. Dans un mode de réalisation, la valeur HLB est de 8 à 15. Dans un mode de réalisation, la valeur HLB est de 8 à 14. Dans un mode de réalisation, la valeur HLB est de 9 à 14. Dans un mode de réalisation, la valeur HLB est de 9 à 13. Dans un mode de réalisation, la valeur HLB est de 8 à 13. Dans un mode de réalisation, la valeur HLB est 8, 9, 10, 11, 12, 13, 14, 15, 16, 17 ou 18. Avantageusement, les compositions E1 et/ou E2 comprennent 2-20% (w/w) dudit émulsifiant hydrosoluble, préférentiellement, 5 à 15% et encore plus préférentiellement, 6 à 12%. Typiquement, l'émulsifiant hydrosoluble est choisi parmi les caséinates, les protéines de blé hydrolysées, préférentiellement, l'agent émulsifiant hydrosoluble est le sodium caséinate.

Dans un mode de réalisation, le sodium caséinate présente une masse moléculaire d'environ 16 000 à environ 25 000 Da. Dans un mode de réalisation, le sodium caséinate présente une masse moléculaire d'environ 19 000 à environ 25 000 Da. Dans un mode de réalisation, le sodium caséinate présente une masse moléculaire d'environ 19 000, environ 20 000, environ 21 000, environ 22 000, environ 23 000, environ 24 000 Da ou environ 25 000. Dans un mode de réalisation, le sodium caséinate présente une masse moléculaire d'environ 20 000 Da.

Avantageusement, la composition H comprend 3-20% (w/w) dudit émulsifiant liposoluble préférentiellement, 5 à 17% et encore plus préférentiellement, 7 à 15%. Typiquement, ledit émulsifiant liposoluble est choisi parmi les monoglycérides, les diglycérides, la lécithine, les esters de sorbitane, les esters de saccharose d'acides gras, les esters de polyglycérol, le polyglycérol polyricinoléique (PGPR) et leurs combinaisons.

L'émulsification est réalisée au moyen d'un homogénéiseur à haute pression ou à système rotor-stator, de manière particulièrement préférée les broyeurs colloïdaux, et les machines dispersantes à dents (type Ultraturax). Aux fins de la présente invention, les systèmes rotor-stator sont des appareils de mélange qui génèrent des contraintes de cisaillement et de poussée élevées au moyen d'une combinaison d'éléments rotatifs et stationnaires. Cette technique permet de disperser uniformément des matières solides (par exemple des charges) ou liquides dans une matrice liquide. Des exemples de tels systèmes rotor-stator sont des moulins à colloïdes, des machines à dispersion dentée et des broyeurs à trois cylindres.

Les appareils de mélange mentionnés sont décrits en détail dans les systèmes Rotor-Stator et Disc Systems for Emulsification Processes; Kai Urban, Gerhard Wagner, David Schaffner, Danny Rdglin, Joachim Ulrich; Génie chimique et technologie, 2006, vol. 29, n ° 1, pages 24 à 31.

Dans un mode de réalisation, la double émulsion comprend en outre des protéines végétales et/ou de la poudre de lactosérum et/ou de la poudre de lait écrémé.

Par « protéines végétales » selon l'invention, on entend des protéines provenant de légumineuses, céréales ou oléagineuses. Parmi les protéines de légumineuses, on choisira de préférence les protéines de soja, d'haricot, de lupin, de luzerne, de fève ou de pois. Parmi les protéines de céréales, on choisira de préférence les protéines de riz, maïs, seigle, blé, avoine, sorgho. Parmi les protéines oléagineuses, on choisira de préférence les protéines colza, tournesol, lin, arachide, Typiquement, les protéines végétales sont choisies parmi des protéines de blé, de pois, de maïs, de pomme-de-terre. Les protéines végétales peuvent être natives ou modifiées notamment hydrolysées telles que par exemple des protéines de légumineuses, céréales ou oléagineuses hydrolysées, on peut citer les protéines de blé hydrolysées.

Par « matière grasse végétale » on entend notamment, de l'huile de palme, l'huile de germe de maïs, l'huile de tournesol, l'huile de colza, l'huile d'olive, l'huile de coprah et leur mélange. Dans un mode de réalisation, la matière grasse végétale est l'huile de colza.

Dans un mode de réalisation, la double émulsion est liquide ou semi-liquide présentant une viscosité de d'environ 10 à environ 200 mPa·s à 25°C.

Dans un mode de réalisation, la double émulsion présente une viscosité de d'environ 10 à environ 100 mPa·s. Dans un mode de réalisation, la double émulsion présente une viscosité de d'environ 55 à environ 100 mPa.s à 25°C.

Dans un mode de réalisation, la double émulsion est liquide présentant une viscosité de d'environ 10 à environ 80 mPa·s. Dans un mode de réalisation, la double émulsion présente une viscosité de d'environ 15 à environ 80 mPa.s. Dans un mode de réalisation, la double émulsion présente une viscosité de d'environ 15 à environ 50 mPa.s. Dans un mode de réalisation, la double émulsion présente une viscosité de d'environ 10 à environ 50 mPa·s. Dans un mode de réalisation, la double émulsion présente une viscosité de d'environ 15 à environ 40 mPa·s. Dans un mode de réalisation, la double émulsion présente une viscosité de d'environ 10 à environ 30 mPa.s. Dans un mode de réalisation, la double émulsion présente une viscosité de d'environ 15 à environ 25 mPa·s. Dans un mode de réalisation, la double émulsion présente une viscosité de d'environ 20 à environ 25 mPa.s. Dans un mode de réalisation, la double émulsion présente une viscosité de d'environ 20 à environ 23 mPa·s. Dans un mode de réalisation, la double émulsion présente une viscosité de d'environ 21 à environ 23 mPa·s à 25°C.

Selon un mode de réalisation, la phase aqueuse interne E1 est dispersée dans la phase huileuse H sous forme de gouttelettes qui présentent un diamètre moyen d'environ 4 à environ 10 microns, de préférence d'environ 6 à environ 8 microns.

L'invention concerne également une composition, notamment une composition de remplacement du lait comprenant une émulsion double E1/H/E2 dans laquelle E1 et E2 sont des phases aqueuses et H est une phase lipidique, et dans laquelle la phase E1 comprend des molécules de glucose.

Selon l'invention, la composition ou la composition de remplacement du lait comprend en outre :

- 2 à 10% de protéines préférentiellement, 3 à 8% encore plus préférentiellement 4 à 6% ; typiquement les protéines sont choisies parmi les protéines végétales telles que par exemple des protéines de pois, de blé, de maïs ou des protéines animales et notamment de lait telles que des caséines ;
- 1 à 14% de lipides, préférentiellement, 1 à 12% encore plus préférentiellement 1 à 5%, typiquement les lipides sont choisis parmi des matières grasses animales et/ou végétales ;
- 3 à 4% de minéraux typiquement choisi parmi le zinc, le magnésium, le sélénium, le chrome, le cuivre et/ou le manganèse.

La double émulsion selon l'invention ou la composition qui la comprend, telle qu'une composition de remplacement du lait est destinée aux mammifères et notamment au jeune bétail ou ruminants.

Selon un premier mode de réalisation, le mammifère est un être humain. Dans un mode de réalisation, l'être humain est intolérant au lactose. Dans un autre mode de réalisation, l'être humain a des troubles métaboliques.

Selon un deuxième mode de réalisation, le mammifère est un animal de compagnie, intolérant au lactose ou non.

Selon un troisième mode de réalisation, l'animal est un bétail. Le bétail comprend, sans limitation, les porcs, les bovins, les chèvres, les cerfs, les moutons, les chevaux et les buffles.

Le terme « ruminants », tel qu'utilisé ici, comprend tout animal saboté élevé dans un milieu agricole qui digère son alimentation avec le procédé de rumination, y compris les animaux néonataux de ruminants qui n'ont pas complètement développé le système des ruminants.

Le terme « jeune », tel qu'utilisé ici, comprend la période de développement d'un animal immature, y compris la période néonatale.

Le terme « néonatal », tel qu'utilisé ici, comprend les six à douze premières semaines de la vie d'un animal.

Dans un mode de réalisation, le mammifère est un jeune bovin ou un bovin néonatal.

L'invention concerne en outre une méthode d'obtention d'une composition de remplacement du lait selon l'invention, ladite méthode comprenant une étape de formation d'une émulsion double E1/H/E2 dans laquelle E1 et E2 sont des phases aqueuses et H est une phase lipidique, et dans laquelle la phase E1 comprend des molécules de glucose.

Typiquement, ladite une émulsion double E1/H/E2 est obtenue par

a) une étape d'obtention d'une composition aqueuse E1 comprenant de 31 à 70%w/w de glucose ;
b) une étape de formation de l'émulsion E1/H, typiquement, cette étape comprend la soumission de la suspension E1/H à une agitation typiquement de 20000 à 30000 tours/minute préférentiellement pendant 3 min ; et
c) une étape de formation de la double émulsion E1/H/E2 typiquement, cette étape comprend une incorporation de l'émulsion E1/H à une composition E2, une étape de soumission de la suspension E1/H/E2 à une agitation typiquement de 7000 à 9000 tours/minute préférentiellement pendant 3min.

La suspension E1/H est obtenue en mélangeant les phases E1 et H dans un ratio (w/w) de 7 :3 à 5 :5, avantageusement 6 :4. La suspension E1/H/E2 est obtenue en mélangeant l'émulsion primaire E1/H et la phase E2 dans un ratio émulsion primaire/ E2 (w/w) de 1 :9 à 8 :2 préférentiellement 2 :8 à 7 :3, typiquement 3 :7 à 4 :6.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

L'invention sera mieux comprise à la lecture des figures et exemples suivants donnés uniquement à titre d'exemple.

## BRÈVE DESCRIPTION DES FIGURES

**Figure 1** est un graphique montrant la cinétique de libération du glucose sans enzyme estimée par le pourcentage de glucose en fonction du temps dans le milieu gastrique puis intestinal de l'émulsion 30/20/50 en comparaison au glucose libre à 12%.

**Figure 2** est un graphique montrant les cinétiques de libération du glucose sans enzyme estimées par le pourcentage de glucose en fonction du temps dans le milieu gastrique puis intestinal des émulsions 12/8/80 (en haut) et 21/14/65 (en bas) en comparaison avec le glucose libre à 4,8 et 8,4% respectivement.

**Figure 3** est un graphique montrant la cinétique de libération du glucose avec enzyme estimée par le pourcentage de glucose en fonction du temps dans le milieu gastrique puis intestinal de l'émulsion 21/14/65 en comparaison au glucose libre à 8,4%.

**EXEMPLES**

La présente invention se comprendra mieux à la lecture des exemples suivants qui illustrent non-limitativement l'invention.

**Matériel et Méthodes**

Pour les émulsions, de l'huile de colza a été utilisée pour la phase huileuse. Du sel 99% de pureté est utilisé pour équilibrer la pression osmotique. De la poudre de lactosérum doux et de la poudre de lait écrémé sont utilisées dans la phase aqueuse externe de l'émulsion industrielle finale. L'émulsifiant hydrophile est du sodium de caséinate (NaCas, MM = 20,000g/mol).

Préparation des émulsions primaires

En se basant sur le travail de Delample et al.(2013), l'émulsion primaire est obtenue en dispersant une phase aqueuse contenant le glucose dans une phase huileuse. La phase aqueuse interne est composée d'un tampon phosphate (Phosphate sodium dibasique dihydrate à pH 12) à 10 mmol/L-1 puis le pH est ajusté à 7,4 à l'aide de solution NaOH ou HCl à 0,5 mol/L-1. A cette phase, 40 % (w/w) de glucose et 0,02 % (w/w) de azide de sodium (SA, inhibiteur microbien) sont ajoutés. La phase aqueuse est préparée à température ambiante. Le PGPR (Polyglycérol Polyricinoléate) est dissout dans l'huile de colza à 70°C. La phase huileuse est composée de 12% (w/w) de ce PGPR qui est un émulsifiant alimentaire dans de l'huile de colza chauffée à 70°C. Cette phase est ensuite refroidie à température ambiante. L'émulsion primaire est préparée en versant progressivement la phase aqueuse primaire (E1) (60 % w/w) dans la phase huile (H) (40 % w/w). Le mélange est porté à l'Ultra-turrax T25 disperser et agité pendant 3 minutes à une vitesse de 22000 tours/minute (rpm) puis l'émulsion est immédiatement utilisée pour préparer l'émulsion double, des échantillons sont aussi conservés à température ambiante pour des analyses ultérieures.

Exemple 1 : Préparation des émulsions doubles E1/H/E2

La phase aqueuse externe (E2) est composée du même tampon phosphate à 10 mmol/L-1 que la phase aqueuse interne et de 8 % (w/w) de caseinate de sodium (émulsion expérimentale). L'émulsion double (finale) est réalisée en ajoutant l'émulsion primaire E1/H à la phase aqueuse externe E2. En effet, 20, 35, 50 % d'émulsion primaire sont ajoutés goutte à goutte à la phase aqueuse externe en agitant à la cuillère puis à l'ultra-turrax pendant une minute à 8000 rpm. De même que la précédente émulsion, des échantillons sont conservés à température ambiante pour des analyses ultérieures. La composition des émulsions primaires et doubles sont représentés dans le tableau 1.

Pour les études de biodisponibilité du glucose, des solutions témoins de glucose sont préparés en fonction de la quantité finale de glucose encapsulé dans l'émulsion double.

Tableau 1 : Formulation des émulsions double E/H/E

| Composition | Concentration | | Ratio |
|---|---|---|---|
| **Emulsion E1/H** | | | |
| **Phase aqueuse interne** | 42 g | | 60%(m/m) |
| Glucose | | 40%(m/m) | |
| Sodium Azide | | 0,02%(m/m) | |
| **Phase huileuse** | 28 g | | 40%(m/m) |
| PGPR | | 12%(m/m) | |
| Huile de Colza | | 88%(m/m) | |
| **Emulsion double E1/H/E2** | | | |
| **Emulsion E1/H** | 18; 31,5; 45 g | | 20, 35, 50 %(m/m) |
| **Phase aqueuse externe** | 72; 58,5; 45 g | | 80, 65, 50%(m/m) |
| Na Caseinates | | 8%(m/m) | |
| Sodium Azide | | 0,04%(m/m) | |

Exemple 2 : Détermination de la taille des gouttelettes d'eau et globules d'huile

La taille des gouttelettes d'eau de l'émulsion primaire contenant 60% de phase aqueuse et 40% de phase huileuse est déterminée en passant l'émulsion dans un appareil de diffusion de la lumière, le TurbiscanLABMa 2000. Cette mesure permet de détecter la déstabilisation des dispersions concentrées à un stade naissant et d'en comprendre les mécanismes pour optimiser les formulations, documenter et accélérer les tests de vieillissement. Cet analyseur macroscopique à balayage vertical permet de faire un balayage complet de la hauteur de l'échantillon et de quantifier à l'aide d'une source de lumière pulsée des phénomènes physiques tels que les flux rétrodiffusés et transmis qui dépendent respectivement des distances moyennes de parcours des photons dans la dispersion : $\lambda^*$ et $\lambda$. Ces paramètres physiques absolus sont directement fonction du diamètre des particules(d) et de la fraction volumique ($\Phi$) des différentes phases. En effet, par la mesure de $\lambda^*$ et $\lambda$, le diamètre moyen des particules de l'échantillon est déterminé.

$$\lambda^* = \left[\frac{2d}{3\Phi(1-g)Qs}\right] \qquad \lambda = \left[\frac{2d}{3\Phi Qs}\right]$$

**g (d) = paramètre d'asymétrie optique**
**Qs (d) = facteur d'efficacité de diffusion**

Les émulsions doubles obtenues sont observées au microscope à épifluorescence inversé. Celui-ci permet de visualiser la taille et la morphologie des globules d'huile et aussi, de visualiser sous forme de masse les gouttelettes d'eau de la phase interne contenue dans les globules d'huile. Cette observation permet ainsi d'évaluer l'efficacité d'émulsification.

Exemple 3 : Analyses rhéologiques des émulsions

Les analyses ont été réalisées à l'aide d'un viscosimètre permettant de faire des mesures des liquides à vitesse imposée. Ce viscosimètre utilise un système de rotor-stator à cylindres co-axiaux, le rotor cisaille le liquide pour déterminer la viscosité apparente de ce dernier. Les paramètres appliqués pour la mesure des échantillons sont comme suit. Une vitesse de cisaillement comprise entre 0,1 et 200s$^{-1}$, un gap de 7,2 mm. La contrainte de cisaillement, $\tau$(Pa) en fonction de la vitesse de cisaillement, $\gamma$(s$^{-1}$) est représentée et la viscosité, $\eta$(Pa.s) est déterminée à température ambiante.
La taille des particules de l'émulsion simple E1/H soit 60/40 qui joue sur la stabilité de l'émulsion double est donc obtenue par mesure au TurbiscanLAB. Elle est estimée à 7,26 $\pm$ 0,50$\mu$m de diamètre. La formation d'un phénomène de floculation est observée dans cette émulsion.
Toutes les émulsions doubles après émulsification sont d'apparence laiteuse ; le liquide obtenu est blanc, fluide, non crémeux et s'écoule facilement.
La contrainte de cisaillements(Pa) par rapport à la vitesse de cisaillement $\gamma$(s$^{-1}$) permet d'avoir le comportement rhéologique des émulsions et de déterminer leur viscosité. Les résultats obtenus montrent que l'émulsion 21/14/65 est de type visqueux Newtonien et que le comportement rhéologique des autres émulsions est identique à celui-ci. Ceci ressort des viscosités des différentes émulsions doubles obtenues à température ambiante qui sont constantes (Tableau 2). Ce résultat est le même pour toutes les formulations d'émulsion.

Tableau 2 : Viscosités des émulsions doubles

|  | Ratio E1/H/E2 | | |
|---|---|---|---|
| Emulsions | 12/8/80 | 21/14/65 | 30/20/50 |
| Viscosité (mPa.s) | 20.87 | 21.60 | 22.46 |

Une observation au microscope optique des différentes émulsions obtenues permet de constater que le protocole d'émulsification permet d'avoir la même taille de particule comprise entre 8 et 30 $\mu$m pour toutes les émulsions testées. La masse représentative des gouttelettes d'eau est aussi visible permettant de confirmer la formation d'une émulsion double.
L'ensemble de ces résultats permet de valider la faisabilité d'une encapsulation du glucose dans une émulsion double eau-dans-huile-dans-eau en deux étapes d'émulsification. La première étape consistant à réaliser une émulsion primaire inverse eau-dans-huile a permis d'obtenir des gouttelettes d'eau d'un diamètre moyen de 7,26 $\pm$0,50$\mu$m après plusieurs essais à différentes vitesses à l'Ultra Turrax. Néanmoins, aucun déphasage au cours du stockage à 4 et 25°C pendant une durée d'un mois n'a été observée entre les deux phases de l'émulsion primaire. La proportion d'émulsifiant lipophile utilisé (soit 12% w/w de PGPR) permet donc la stabilité de cette émulsion. Il est important que l'émulsifiant lipophile soit en excès dans la phase huileuse pour une meilleur stabilisation. A priori, la quantité ajoutée ici est suffisante pour la stabilité de l'émulsion primaire.

La seconde étape est la dispersion douce de l'émulsion primaire dans la phase aqueuse externe. Les images obtenues montrent des globules d'huile sphériques contenant les gouttelettes d'eau (observées sous forme de masse). La vitesse d'homogénéisation à l'Ultra Turrax permet d'avoir de globules d'huile d'une taille comprise entre 8 et 30 $\mu$m. Un phénomène d'instabilité est cependant observé dans les émulsions doubles après 24 heures de stockage à 4 et 25°C. En effet, la différence de densité entre les globules et la phase continue entraine le crémage des globules d'huile. Ce phénomène peut conduire à la transformation au cours du temps de l'émulsion double E/H/E en émulsion simple E/H. Il apparait que l'utilisation de Na caséinates à 8 et 10% (w/w) permet d'obtenir une meilleure conservation de l'émulsion dans le temps.

Exemple 4 : Essais de biodisponibilité *in vitro* du glucose encapsulé

L'essai de libération du glucose par dialyse permet d'évaluer l'efficacité d'encapsulation du glucose en se basant sur une dialyse. La méthode utilisée est celle décrite par Makarram et al.(2009). Deux milieux simulant (avec et sans enzyme) le milieu gastrique et le milieu intestinal des jeunes bovins sont mis en place pour le test de biodisponibilité, la libération du glucose encapsulé se fait en batch. 24 heures après la préparation de l'émulsion double, 25 g d'émulsion double ou de solution témoin de glucose sont pesés et mis dans une membrane de dialyse de taille 35 MM permettant le passage de molécules d'environ 10 kDa. La taille des pores de membranes de dialyse a été choisie de telle sorte à faire une sélection entre les molécules de glucose et celles de protéines ou de lipides par rapport à leur poids moléculaire. Ces 25 grammes constituent le liquide de dialyse ; la membrane est ensuite immergée dans 100 grammes de milieu gastrique pendant environ 2 heures à 37°C sous agitation lente de 100 rpm. Les 2 heures correspondent au temps de séjour estimé de l'émulsion dans l'œsophage avant l'atteinte de la caillette. La membrane de dialyse est ensuite retirée du milieu gastrique et immergée dans le milieu intestinal pour une durée supérieure à 3 heures. Des prélèvements en duplicata de 500 $\mu$l de milieu de contre dialyse sont effectués toutes les 30 minutes et sont immédiatement remplacés par du milieu gastrique ou intestinal, puis le glucose est dosé avec un kit de dosage Glucose RTU ™ (Biomérieux).

Le milieu gastrique sans enzyme à pH 1,5 est composé de 0,08 mol.L-1 d'acide chlorhydrique contenant 0,2 % d'hydroxyde de sodium; avec enzyme, 3 g/L de pepsine de la muqueuse de l'estomac du porc (Sigma-Aldrich) est rajouté au milieu et le pH est ajusté à 1,8. Le choix du pH à 1,8 pour le milieu est dû à l'enzyme qui a une activité maximale entre pH 1,7 et 3. Le milieu intestinal est constitué d'une solution de 0,05 mol.L-1 de tampon phosphate à pH 7,25 pour le milieu sans enzyme, et avec enzyme, 10 g/L de pancréatine sont ajoutés et le pH est de nouveau ajusté à 6,5.

A partir de la concentration en g/L de glucose, le pourcentage de libération du glucose est calculé selon les formules suivantes :

$$\% \text{ glucose} = \frac{[\text{Glucose}]1 \times \text{VM}}{\text{Masse glucose}} \times 100 \quad (1)$$

$$\% \text{ glucose} = \frac{([\text{Glucose}]2 \times \text{VM}) + ([\text{Glucose}]1 \times \text{VPE})}{\text{Masse glucose}} \times 100 \quad (2)$$

[Glucose] 1= concentration de glucose obtenue aux 30 premières minute
[Glucose] 2 ou n+1 = concentration de glucose au cours de l'étude
VM = volume du milieu gastrique ou intestinal, soit 100 mL.
VPE = volume prélevé pour le dosage, soit 500 $\mu$L

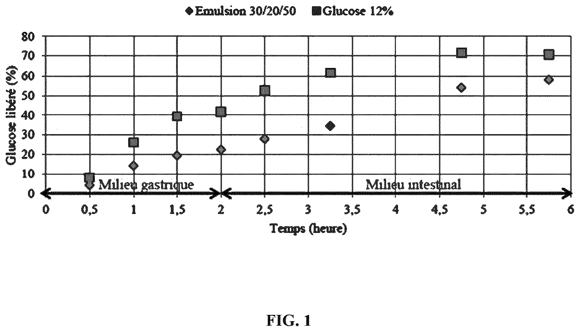

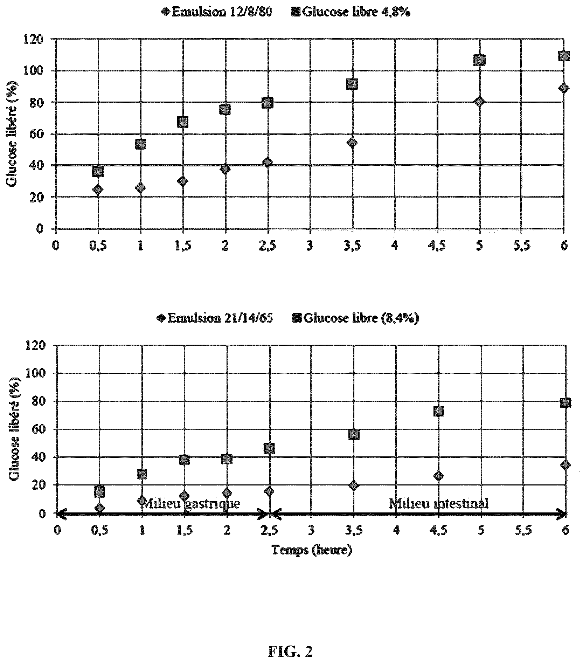

Les figures 1 et 2 présentent les cinétiques de libération du glucose libre et encapsulé en batch dans le milieu gastrique puis dans le milieu intestinal pour différentes formulations d'émulsion. Par exemple pour l'émulsion 30/20/50 et le glucose libre à 12%, les résultats montrent une libération dans le milieu gastrique de 20% de glucose contre 40% au bout de 2 heures, respectivement pour l'émulsion et le glucose libre. Après 5,75 heures, la libération atteint 70% pour le glucose libre contre environ 59 % pour l'émulsion 30/20/50 (Figure 1). Le phénomène observé avec l'émulsion 30/20/50 par rapport au témoin est le même dans les émulsions de compositions différentes 21/14/65 et 12/8/20 (Figure 2).

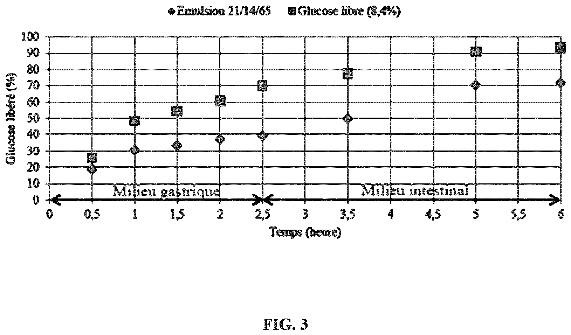

L'émulsion 21/14/65 a été choisie pour tester la libération du glucose en présence de pepsine dans le jus gastrique et de pancréatine dans le jus intestinal, sa cinétique de libération est représentée sur la figure 3. Les résultats montrent des comportements identiques au milieu sans enzyme. Le pourcentage de glucose libéré atteint environ 70 % pour le glucose encapsulé et près de 95 % pour le glucose non encapsulé en 6 heures d'incubation en batch dans les deux milieux.

Les exemples ci-dessus montrent une différence significative de libération de glucose encapsulé par l'utilisation des émulsions proposées comparativement au glucose libre. L'encapsulation du glucose par une double émulsion de la présente invention est donc une bonne solution pour la substitution du lactose par le glucose, notamment dans les compositions de remplacement du lait.

## Revendications

1. Double émulsion eau-huile-eau, comprenant :

   - une phase aqueuse interne E1, comprenant du glucose de 31 à 70% w/w, de préférence de 35 à 55% w/w en poids par rapport à la masse d'E1 ;
   - une phase huileuse H, comprenant une matière grasse animale et/ou végétale, et un émulsifiant lipophile ; le ratio massique de la phase aqueuse interne E1 sur la phase huileuse H étant de 7 : 3 à 5 : 5 ; et
   - une phase aqueuse externe E2, comprenant un émulsifiant hydrosoluble.

2. Double émulsion selon la revendication **1,** dans laquelle l'émulsifiant hydrosoluble est choisi parmi les caséinates, les protéines de blé hydrolysées, préférentiellement, l'agent émulsifiant hydrosoluble est le sodium caséinate.

3. Double émulsion selon la revendication **1** ou la revendication **2,** dans laquelle l'émulsifiant hydrosoluble est de 6% à 12% w/w, de préférence de 8 à 10% w/w en poids par rapport à la masse de la phase externe E2.

4. Double émulsion selon l'une quelconque des revendications **1** à **3,** dans laquelle l'émulsifiant lipophile est choisi parmi les monoglycérides, les diglycérides, la lécithine, les esters de sorbitane, les esters de saccharose d'acides gras, les esters de polyglycérol, le polyglycérol polyricinoléate et leurs combinaisons ; de préférence l'émulsifiant lipophile est le polyglycérol polyricinoléate.

5. Double émulsion selon l'une quelconque des revendications **1** à **4,** dans laquelle la matière grasse de la phase huileuse H est de la matière grasse végétale choisie parmi de l'huile de palme, l'huile de germe de maïs, l'huile de tournesol, l'huile de colza, l'huile d'olive, l'huile de coprah et leur mélange.

6. Double émulsion selon l'une quelconque des revendications **1** à **5,** dans laquelle la phase aqueuse extérieure E2 comprend en outre des protéines végétales choisies parmi des protéines de légumineuses, céréales et/ou oléagineuses ; et/ou des protéines végétales hydrolysées, préférentiellement des protéines de blé hydrolysées.

7. Double émulsion selon l'une quelconque des revendications **1** à **6,** dans laquelle la phase aqueuse extérieure E2 comprend en outre la poudre de lactosérum doux et/ou de la poudre de lait écrémé.

8. Double émulsion selon l'une quelconque des revendications **1** à **7,** ladite double émulsion étant liquide, présentant une viscosité d'environ 10 à environ 100 mPa.s à 25°C.

9. Double émulsion selon l'une quelconque des revendications **1** à **8,** dans laquelle la phase aqueuse interne E1 est dispersée dans la phase huileuse H sous forme de gouttelettes, lesquelles gouttelettes présentent un diamètre moyen d'environ 4 à environ 10 microns, de préférence d'environ 6 à environ 8 microns.

10. Composition comprenant la double émulsion selon l'une quelconque des revendications **1** à **9.**

11. Composition selon la revendication **10,** ladite composition comprend :

    - des protéines, de préférence des protéines végétales et/ou animales, dans une concentration de 2 à 10 % w/w par rapport au poids de la composition ;
    - des lipides, préférentiellement choisis parmi des matières grasses animales et/ou végétales, dans une concentration de 1 à 14 % w/w par rapport au poids de la composition ; et
    - des minéraux, dans une concentration de 3 à 4 % w/w par rapport au poids de la composition.

12. Composition selon la revendication **11,** dans laquelle les lipides sont dans une concentration de 1 à 5 % w/w par rapport au poids de la composition.

13. Composition selon l'une quelconque des revendications **10** à **12,** pour son utilisation en tant que composition nutritionnelle pour un animal, de préférence un mammifère, préférentiellement un ruminant, plus préférentiellement un ruminant néonatal, encore plus préférentiellement un bovin néonatal.

14. Utilisation d'une composition telle que décrite dans l'une quelconque des revendications **10** à **13** en tant que composition nutritionnelle pour un animal, de préférence un mammifère, préférentiellement un ruminant, plus préférentiellement un ruminant néonatal, encore plus préférentiellement un bovin néonatal.

15. Procédé de préparation d'une double émulsion selon l'une quelconque des revendications **1** à **9** ou de la composition selon l'une quelconque des revendications **10** à **13**, ledit procédé comprenant les étapes de :

a) préparer une phase aqueuse E1, ladite phase aqueuse E1 comprenant du glucose de 31 à 70% w/w, de préférence de 35 à 55% w/w en poids par rapport à la masse d'E1 ;
b) préparer une émulsion primaire E1/H, ladite émulsion primaire E1/H comprend la phase aqueuse E1 obtenue à l'étape (a), avec une phase huileuse H, ladite phase huileuse H comprenant une matière grasse animale et/ou végétale, et un émulsifiant lipophile; de préférence la préparation de l'émulsion primaire E1/H comprend la soumission de l'émulsion E1/H à une agitation de 20000 à 30000 tours/minute ; et
c) incorporer l'émulsion primaire E1/H obtenue à l'étape (b) dans une phase aqueuse E2, ladite phase aqueuse E2 comprenant un émulsifiant hydrosoluble.

**Patentansprüche**

1. Wasser-Öl-Wasser-Doppelemulsion, umfassend:

- eine innere wässrige Phase E1, umfassend 31 bis 70 Gew.-% w/w, bevorzugt 35 bis 55 Gew.-% w/w Glucose in Bezug auf die Masse von E1;
- eine ölige Phase H, umfassend ein tierisches und/oder pflanzliches Fett und einen lipophilen Emulgator; wobei das Massenverhältnis der inneren wässrigen Phase E1 zur öligen Phase H von 7 : 3 bis 5 : 5 ist; und
- eine äußere wässrige Phase E2, umfassend einen wasserlöslichen Emulgator.

2. Doppelemulsion nach Anspruch **1,** wobei der wasserlösliche Emulgator aus den Kaseinaten, den hydrolysierten Weizenproteinen ausgewählt ist, wobei das wasserlösliche Emulgatormittel vorzugsweise Natriumkaseinat ist.

3. Doppelemulsion nach Anspruch **1** oder Anspruch **2,** wobei der wasserlösliche Emulgator 6 % bis 12 Gew.-% w/w, bevorzugt 8 bis 10 Gew.-% w/w in Bezug auf die Masse der äußeren Phase E2 beträgt.

4. Doppelemulsion nach einem der Ansprüche **1** bis **3,** wobei der lipophile Emulgator aus den Monogloceriden, den Diglyceriden, dem Lecithin, den Sorbitanestern, den Saccharosefettsäureestern, den Polyglycerolestern, dem Polyglycerol-Polyricinoleat und deren Kombinationen ausgewählt ist; wobei der lipophile Emulgator bevorzugt das Polyglycerol-Polyricinoleat ist.

5. Doppelemulsion nach einem der Ansprüche **1** bis **4,** wobei das Fett der öligen Phase H Pflanzenfett ist, ausgewählt aus dem Palmöl, dem Maiskeimöl, dem Sonnenblumenöl, dem Rapsöl, dem Olivenöl, dem Kopraöl und deren Gemischen.

6. Doppelemulsion nach einem der Ansprüche **1** bis **5,** wobei die äußere wässrige Phase E2 ferner Pflanzenproteine umfasst, ausgewählt aus den Proteinen von Leguminosen, Getreiden und/oder Ölpflanzen; und/oder hydrolysierte Pflanzenproteine, vorzugsweise hydrolysierte Weizenproteine.

7. Doppelemulsion nach einem der Ansprüche **1** bis **6,** wobei die äußere wässrige Phase E2 ferner Süßmolkenpulver und/oder Magermilchpulver umfasst.

8. Doppelemulsion nach einem der Ansprüche **1** bis **7,** wobei die Doppelemulsion, die flüssig ist, eine Viskosität von zirka 10 bis zirka 100 mPa.s bei 25 °C aufweist.

9. Doppelemulsion nach einem der Ansprüche **1** bis **8,** wobei die innere wässrige Phase E1 in der öligen Phase H in Form von Tröpfchen dispergiert ist, wobei die Tröpfchen einen mittleren Durchmesser von zirka 4 bis zirka 10 Mikron, bevorzugt von zirka 6 bis zirka 8 Mikron aufweisen.

10. Zusammensetzung, umfassend die Doppelemulsion nach einem der Ansprüche **1** bis **9.**

11. Zusammensetzung nach Anspruch **10,** wobei die Zusammensetzung umfasst:

   - Proteine, bevorzugt pflanzliche und/oder tierische Proteine, in einer Konzentration von 2 bis 10 % w/w in Bezug auf das Gewicht der Zusammensetzung;
   - Lipide, vorzugsweise ausgewählt aus den tierischen und/oder pflanzlichen Fetten in einer Konzentration von 1 bis 14 % w/w in Bezug auf das Gewicht der Zusammensetzung; und
   - Mineralien in einer Konzentration von 3 bis 4 % w/w in Bezug auf das Gewicht der Zusammensetzung.

12. Zusammensetzung nach Anspruch **11,** wobei die Lipide in einer Konzentration von 1 bis 5 % w/w in Bezug auf das Gewicht der Zusammensetzung sind.

13. Zusammensetzung nach einem der Ansprüche **10** bis **12** für ihre Verwendung als Ernährungszusammensetzung für ein Tier, bevorzugt ein Säugetier, vorzugsweise einen Wiederkäuer, vorzugsweiser einen neonatalen Wiederkäuer, noch vorzugsweiser ein neonatales Rind.

14. Verwendung einer Zusammensetzung wie in einem der Ansprüche **10** bis **13** beschrieben als Ernährungszusammensetzung für ein Tier, bevorzugt ein Säugetier, vorzugsweise einen Wiederkäuer, vorzugsweiser einen neonatalen Wiederkäuer, noch vorzugsweiser ein neonatales Rind.

15. Verfahren zur Herstellung einer Doppelemulsion nach einem der Ansprüche **1** bis **9** oder der Zusammensetzung nach einem der Ansprüche **10** bis **13,** wobei das Verfahren die folgenden Schritte umfasst:

   a) Herstellen eine wässrigen Phase E1, wobei die wässrige Phase E1 31 bis 70 Gew.-% w/w, bevorzugt 35 bis 55 Gew.-% w/w Glucose in Bezug auf die Masse von E1 umfasst;
   b) Herstellen einer Primäremulsion E1/H, wobei die Primäremulsion E1/H die in Schritt (a) erhaltene wässrige Phase umfasst, mit einer öligen Phase H, wobei die ölige Phase H ein tierisches und/oder pflanzliches Fett und einen lipophilen Emulgator umfasst; wobei bevorzugt die Herstellung der Primäremulsion E1/H ein Rühren der Emulsion E1/H bei 20000 bis 300000 Umdrehungen/Minute umfasst; und
   c) Einarbeiten der in Schritt (b) erhaltenen Primäremulsion E1/H in eine wässrige Phase E2, wobei die wässrige Phase einen wasserlöslichen Emulgator umfasst.

**Claims**

1. Double water-oil-water emulsion, comprising:

   - an internal aqueous phase E1, comprising glucose from 31 to 70% w/w, preferably from 35 to 55% w/w by weight relative to the mass of E1;
   - an oily phase H, comprising an animal and/or vegetable fat, and a lipophilic emulsifier; the mass ratio of the internal aqueous phase E1 on the oily phase H being from 7:3 to 5:5; and
   - an external aqueous phase E2, comprising a water-soluble emulsifier.

2. Double emulsion according to claim **1,** wherein the water-soluble emulsifier is selected from caseinates, hydrolysed wheat proteins, preferably the water-soluble emulsifier is sodium caseinate.

3. Double emulsion according to claim **1** or claim **2,** wherein the water-soluble emulsifier is 6% to 12% w/w, preferably 8 to 10% w/w by weight relative to the mass of the external phase E2.

4. Double emulsion according to any one of claims **1** to **3,** wherein the lipophilic emulsifier is selected from monoglycerides, diglycerides, lecithin, sorbitan esters, sucrose esters of fatty acids, esters of polyglycerol, polyglycerol polyricinoleate and combinations thereof; preferably the lipophilic emulsifier is polyglycerol polyricinoleate.

5. Double emulsion according to any one of claims **1** to **4,** wherein the fat of the oily phase H is vegetable fat selected from palm oil, corn germ oil, sunflower oil, rapeseed oil, olive oil, coconut oil and their mixture.

6. Double emulsion according to any one of claims **1** to **5,** wherein the outer aqueous phase E2 further comprises vegetable proteins selected from proteins from legumes, cereals and/or oilseeds; and/or hydrolysed vegetable proteins, preferably hydrolysed wheat proteins.

7. Double emulsion according to any one of claims **1** to **6,** wherein the outer aqueous phase E2 further comprises sweet whey powder and/or skimmed milk powder.

8. Double emulsion according to any one of claims **1** to **7,** said double emulsion being liquid, having a viscosity from about 10 to about 100 mPa.s at 25 °C.

9. Double emulsion according to any one of claims **1** to **8,** wherein the internal aqueous phase E1 is dispersed in the oily phase H in the form of droplets, said droplets have an average diameter from about 4 to about 10 microns, preferably from about 6 to about 8 microns.

10. Composition comprising the double emulsion according to any one of claims **1** to **9.**

11. The composition of claim **10,** said composition comprising:

   - proteins, preferably plant and/or animal proteins, in a concentration from 2 to 10% w/w relative to the weight of the composition;
   - lipids, preferably selected from animal and/or vegetable fats, in a concentration from 1 to 14% w/w relative to the weight of the composition; and
   - minerals, in a concentration from 3 to 4% w/w relative to the weight of the composition.

12. The composition of claim **11,** wherein the lipids are in a concentration from 1 to 5% w/w relative to the weight of the composition.

13. Composition according to any one of claims **10** to **12,** for use as a nutritional composition for an animal, preferably a mammal, preferably a ruminant, more preferably a neonatal ruminant, even more preferably a neonatal bovine.

14. Use of a composition as described in any one of claims **10** to **13** as a nutritional composition for an animal, preferably a mammal, preferably a ruminant, more preferably a neonatal ruminant, even more preferably a neonatal bovine.

15. Process for preparing a double emulsion according to any one of claims **1** to **9** or the composition according to any one of claims **10** to **13,** said process comprising the steps of:

   a) preparing an aqueous phase E1, said aqueous phase E1 comprising glucose from 31 to 70% w/w, preferably from 35 to 55% w/w by weight relative to the mass of E1;
   b) preparing a primary emulsion E1/H, said primary emulsion E1/H comprising the aqueous phase E1 obtained in step (a), with an oily phase H, said oily phase H comprising an animal and/or vegetable fat, and a lipophilic emulsifier; preferably the preparation of the primary E1/H emulsion comprises submitting the E1/H emulsion to stirring from 20,000 to 30,000 revolutions/minute; and
   c) incorporate the primary emulsion E1/H obtained in step (b) in an aqueous phase E2, said aqueous phase E2 comprising a water-soluble emulsifier.

FIG. 1

FIG. 2

FIG. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2828378 A1

- WO 2015197089 A1

**Littérature non-brevet citée dans la description**

- **KAI URBAN ; GERHARD WAGNER ; DAVID SCHAFFNER ; DANNY RDGLIN ; JOACHIM ULRICH.** Rotor-Stator et Disc Systems for Emulsification Processes. *Génie chimique et technologie,* 2006, vol. 29 (1), 24-31